Europäisches Patentamt

European Patent Office

Office européen des brevets

(11) Publication number: **0 088 007**

**B1**

(12) **EUROPEAN PATENT SPECIFICATION**

(45) Date of publication of patent specification: **12.11.86**

(51) Int. Cl.⁴: **C 12 P 33/00, C 07 J 1/00**

(21) Application number: **83400341.0**

(22) Date of filing: **18.02.83**

(54) Microbial process for producing 12-hydroxyandrosta-1,4-diene-3,17-dione.

(30) Priority: **26.02.82 JP 30898/82**
**28.10.82 JP 190571/82**

(43) Date of publication of application:
**07.09.83 Bulletin 83/36**

(45) Publication of the grant of the patent:
**12.11.86 Bulletin 86/46**

(84) Designated Contracting States:
**FR IT NL**

(56) References cited:
**WO-A-81/02427**

**TETRAHEDRON, vol. 37, no. 9, May 1981, pages 1747-1751, Pergamon Press Ltd., Oxford, GB. R.A. LEPPIK: "Deoxycholic acid degradation by a pseudomonas sp: phenolic and neutral products"**

**TETRAHEDRON, vol. 32, no. 1, January 1976, pages 89-93, Pergamon Press, Oxford, GB. P.J. BARNES et al.: "Degradation of deoxycholic acid by pseudomonas Sp. NCIB 10590"**

(73) Proprietor: **KURARAY CO., LTD.**
**1621 Sakazu**
**Kurashiki-City Okayama Prefecture (JP)**

(72) Inventor: **Tsuji, Masao**
**1625, Sakazu**
**Kurashiki-shi Okayama-ken (JP)**
Inventor: **Bunno, Masayasu House No. 22-501**
**Edogawadai-Odakyu-Haitsu 5-1 Fujimidai 2-chome**
**Nagareyama-shi Chiba-ken (JP)**
Inventor: **Harada, Hidemi**
**1621, Sakazu**
**Kurashiki-shi Okayama-ken (JP)**
Inventor: **Sugiura, Tsutomu**
**1625, Sakazu**
**Kurashiki-shi Okayama-ken (JP)**
Inventor: **Ichihara, Yoshihiro**
**1652, Sakazu**
**Kurashiki-shi Okayama-ken (JP)**

(74) Representative: **Descourtieux, Philippe et al**
**CABINET BEAU de LOMENIE 55 rue d'Amsterdam**
**F-75008 Paris (FR)**

Courier Press, Leamington Spa, England.

## Description

The present invention relates to a microbial process for producing 12 - hydroxyandrosta - 1,4 - dien - 3,17 - dione from deoxycholic acid or a salt thereof. More particularly, according to the present invention, 12α - hydroxyandrosta - 1,4 - dien - 3,17 - dione or 12β - hydroxyandrosta - 1,4 - dien - 3,17 - dione can be produced in a high yield by cultivating a certain microbe of the species *Pseudomonas putida* in a culture medium containing deoxycholic acid or a salt thereof as a substrate.

12-Hydroxyandrosta-1,4-dien-3,17-dione can be used as a starting material for the production of 4-androsten-3,17-dione which is a useful starting material for the synthesis of steroid diuretics.

Several microbial processes for producing 12α-hydroxyandrosta-1,4-dien-3,17-dione (hereinafter referred to as 12α-hydroxy ADD) or 12β-hydroxyandrosta-1,4-dien-3,17-dione (hereinafter referred to as 12β-hydroxy ADD) have been known heretofore in the prior art. For example, P. J. Barnes et al. disclose a process using *Pseudomonas sp.* NCIB 10590 strain [J. Chem. Soc. Chem. Commum. (1974), pp 115—116; and Tetrahedron, *32*, pp 89—93 (1976)]. In this process, *Pseudomonas sp.* NCIB 10590 strain is cultivated in a mineral medium (10 liters) containing 1.0 g/l of sodium deoxycholate as a substrate for 14 hours under aerobic conditions to produce as major products 12β-hydroxy ADD (960 mg) and 12 - hydroxypregna - 1,4 - dien - 3 - one - 20α - carboxylic acid (657 mg) together with as minor products 12α-hydroxy ADD, 12β - hydroxy - 4 - androsten - 3,17 - dione and 12ξ, 17ξ - dihydroxy - 4 - androsten - 3 - one. However, this process is impractical because 12α-hydroxy ADD is obtained as a mere minor product; the yield of 12β-hydroxy ADD is very low (less than 10 %); 12α - hydroxypregna - 1,4 - dien - 3 - one - 20α - carboxylic acid obtained as a by-product amounts to about 2/3 time of the yield of 12β-hydroxy ADD and the selectivity for 12β-hydroxy ADD is low; and the concentration of the substrate is low (0.1 %). Besides, as to *Pseudomonas sp.* NCIB 10590 strain used in this process, there is no disclosure other than that the strain has been isolated from animal faeces and belongs to the genus *Pseudomonas.*

R. A. Leppik discloses a process using *Pseudomonas sp.* MR 108 strain [Tetrahedron, *37*, pp 1747—1751 (1981)]. In this process, *Pseudomonas sp.* MR 108 strain is cultivated in a mineral salts medium (15 liters) containing 2.0 g/l of deoxycholic acid as a substrate under aerobic conditions to produce 12β-hydroxy ADD (270 mg), 12α-hydroxy ADD (146) mg), 3,12β - dihydroxy - 9,10 - secoandrosta - 1,3,5(10) - trien - 9,17 - dione (29 mg) and 3aα - H - 4α - [3' - propionic acid] - 5α - hydroxy - 7aβ - methylhexahydro - 1 - indanone - δ - lactone (34 mg). However, this process can not be employed in the industrial production of 12-hydroxy ADD because the total yield of 12α-hydroxy ADD and 12β-hydroxy ADD is low (less than 2 %); and the concentration of the substrate is low (0.2 %). Besides, as to *Pseudomonas sp.* MR 108 strain used in this process, there is no disclosure other than that the strain has been isolated from soil and belongs to the genus *Pseudomonas.*

Further, M. E. Tenneson et al. disclose a process for producing 12β-hydroxy ADD and 12α - hydroxypregna - 1,4 - dien - 3 - one - 20α - carboxylic acid from deoxycholic acid by using an anaerobic strain of *Escherichia coli [Biochem. Soc. Trans., 5,* pp 1758—1760 (1977)]. R. W. Owen et al. disclose a process for producing 12α-hydroxy ADD, 12β-hydroxy ADD, 12β - hydroxy - 4 - androsten - 3,17 - dione and 12α - hydroxypregna - 1,4 - dien - 3 - one - 20α - carboxylic acid from deoxycholic acid by using *Bacteroides fragilis* XF 23 strain or *Bacteroides fragilis subsp. thetaiotaomicron* E 59 strain [Biochem. Soc. Trans., *5,* pp 1711—1713 (1977)]. However, in these processes, anaerobic strains are used and hence, where these processes are applied to the industrial production of 12-hydroxy ADD, there are such problems that a low concentration of the substrate and a long-term cultivation are unavoidable and a yield of the product is low.

The present inventors have intensively searched and studied in order to obtain a microbe which is useful in the industrial production of 12-hydroxy ADD by utilizing deoxycholic acid or a salt thereof as a substrate. As the result, it has been found that a certain microbe of the species *Pseudomonas putida* can produce 12-hydroxy ADD from deoxycholic acid or a salt thereof in a high selectivity and a high yield.

The main object of the present invention is to provide a microbial process for producing 12-hydroxy ADD, i.e. 12α-hydroxy ADD or 12β-hydroxy ADD, which is suitable for the industrial production of said compound. This object as well as other objects and advantages of the present invention will become apparent to those skilled in the art from the following description.

According to the present invention, there is provided a microbial process for producing 12-hydroxy ADD which comprises cultivating a microbe of the species *Pseudomonas putida,* which is capable or producing 12-hydroxy ADD by utilizing deoxycholic acid or a salt thereof as a substrate, in a culture medium containing the substrate to produce 12-hydroxy ADD and collecting the product.

The microbes to be used in the present invention may be wild-type strains of the species *Pseudomonas putida* or mutants thereof obtained by natural mutation or a conventional mutagenic treatment such as X-ray irradiation, ultraviolet irradiation, treatment with a chemical mutagen such as N - methyl - N' - nitro - N - nitrosoguanidine, 4 - nitro - quinoline - N - oxide, acriflavine or ethylmethane sulfonate or a combination thereof and the like.

Among the microbes being capable of producing 12-hydroxy ADD by utilizing deoxycholic acid or a salt thereof as a substrate which are obtained by the present inventors, the representatives have been deposited with Fermentation Research Institute, Agency of Industrial Science and Technology, Japan on February 5, 1982 hereinafter, referred to as FERM). They are *Pseudomonas putida* D 4014 strain (FERM

2

**0 088 007**

BP-205 of February 5, 1982 and *Pseudomonas putida* D4014—A1099 strain (FERM BP-207 of February 5, 1982, *Pseudomonas putida* D4014 strain is a wild-type strain isolated from soil and *Pseudomonas putida* D4014—A1099 strain is a mutant thereof. Particularly, *Pseudomonas putida* D4014 strain is useful for the production of 12β-hydroxy ADD and *Pseudomonas putida* D4014—A1099 strain is useful for the production of 12α-hydroxy ADD. The morphological, cultural and physiological characteristics of these strains are shown in Table 1.

TABLE 1

| Characteristics | Ps. putida D4014 | Ps. putida D4014—A1099 |
|---|---|---|
| Microscopic observation | | |
| Form | rods | rods |
| Size (μ) | 0.5—0.6×1.5—2.9 | 0.5×1.5—2.5 |
| Flagellum | polar flagella | polar flagella |
| Spore | nil | nil |
| Gram stain | negative | negative |
| Acid fast stain | nil | nil |
| Cultural observation | | |
| Bouillon agar plate culture | circular, raised, convex, smooth, entire | circular, raised, convex, smooth, entire |
| Bouillon agar slant culture | moderate growth, filiform, translucent, fluorescent | moderate growth, filiform, translucent, fluorescent |
| Bouillon broth | turbid, pellicle | turbid |
| Growth temperature | growth at 37°C | growth at 37°C |
| Gelatin stab | no liquefaction | no liquefaction |
| Litmus milk | alkaline, milk unchanged | alkaline, milk unchanged |
| BCP milk | alkaline, milk unchanged | alkaline, milk unchanged |
| Physiological character[1] | | |
| Nitrate reduction | − | − |
| Denitrification | − | − |
| Methyl red test | + | + |
| Voges-Proskauer test | − | − |
| Indole production | − | − |
| $H_2S$ production | − | − |
| Starch hydrolysis | − | − |
| Citrate utilization | + | + |
| Assimilation of inorganic nitrogen sources | + | + |
| Urease | ± | ± |
| Oxidase | + | + |

3

TABLE 1 (contd.)

| Characteristics | Ps. putida D4014 | | Ps. putida D4014—A1099 | |
|---|---|---|---|---|
| Catalase | + | | + | |
| Arginine dihydrolase | + | | + | |
| Require of oxygen | aerobic | | aerobic | |
| Oxidation/Fermentation test | oxidative | | oxidative | |
| Production of acids and gases[2] from carbohydrates | acids | gases | acids | gases |
| L-Arabinose | + | — | + | — |
| D-Xylose | + | — | + | — |
| D-Glucose | + | — | + | — |
| D-Mannose | + | — | + | — |
| D-Fructose | — | — | — | — |
| D-Galactose | + | — | + | — |
| Maltose | — | — | — | — |
| Sucrose | — | — | — | — |
| Lactose | — | — | — | — |
| Trehalose | — | — | — | — |
| D-Sorbitol | — | — | — | — |
| D-Mannitol | — | — | — | — |
| Inositol | — | — | — | — |
| Glycerol | — | — | — | — |
| Starch | — | — | — | — |

Remarks:

[1] The symbols used in Physiological character have the following meaning.

+: The strain has the corresponding character or produces the corresponding product.

±: It is difficult to determine whether or not the strain has the corresponding character or produces the corresponding product.

—: The strain does not have the corresponding character or does not produce the corresponding product.

[2] By using Hugh and Leifson medium in which each of the carbohydrates shown in Table 1 was substituted for the carbon source thereof, production of acids and gases by the strain was observed.

+: An acid or a gas is produced.

±: It is difficult to determine whether or not an acid or a gas is produced.

—: An acid or a gas is not produced.

On the basis of these morphological, cultural and physiological characteristics, the classification of the strains has been determined according to Bergey's Manual of Determinative Bacteriology 7th and 8th Editions.

It is determined that *Pseudomonas putida* D4014 strain is a microbe of the genus *Pseudomonas* in view of its microscopic observation such as rod-form, polar flagella and negative in gram strain as well as its physiological character such as positive in both oxidase and catalase reactions, aerobic and oxidative in Oxidation/Fermentation test. Further, it is determined that *Pseudomonas putida* D4014 strain is a microbe of the species *Pseudomonas putida* in view of fluorescent in cultural observation, no liquefaction of gelatin stab, growth at 37°C, production of arginine dihydrolase and the like. It is determined that *Pseudomonas putida* D4014—A1099 strain is a microbe of the species *Pseudomonas putida* because, in general, a mutant is classified into the same species of its parent strain.

The process of the present invention is carried out by cultivating a microbe of the species *Pseudomonas putida,* which is capable of producing 12-hydroxy ADD by utilizing deoxycholic acid of a salt thereof as a substrate, in a culture medium containing the substrate to produce 12-hydroxy ADD and collecting the resulting 12-hydroxy ADD.

In the present invention, deoxycholic acid per se can be used as a substrate. There can be also used an alkali metal salt of deoxycholic acid such as sodium deoxycholate, potassium deoxycholate or the like. When a deoxycholate is used, it is dissolved in water to prepare an aqueous solution containing the deoxycholate in a predetermined concentration. Alternatively, a certain amount of an alkali metal compound which forms a salt with deoxycholic acid may previously be dissolved in water and added thereto deoxycholic acid to obtain an aqueous solution containing a deoxycholate in a predetermined concentration. In general, a concentration of the substrate in a culture medium may be varied widely in a range of from about 1 to 100 g/l as deoxycholic acid. However, in view of a yield of the desired product, conditions for cultivation and economic efficiency such as operability, workability and the like, it is preferable to use the substrate in a concentration of about 2 to 50 g/l as deoxycholic acid.

Cultivation can be carried out according to a known method under aerobic conditions and, usually, a shaking or submerged culture using a liquid medium is employed.

As the medium, there can be used one containing nutrients which can be assimilated by the microbe to be used. The medium can contain deoxycholic acid or a salt thereof as the sole carbon source. However, it is preferable to use deoxycholic acid or a salt thereof together with an additional carbon source such as glucose, glycerol, peptone, meat extract, malt extract, yeast extract or a mixture thereof. Usually, the additional carbon source can be added to the medium in a concentration of about 0.1 to 20 g/l. As a nitrogen source, there can be used an inorganic nitrogen source such as ammonium sulfate, ammonium chloride, ammonium phosphate, ammonium nitrate, sodium nitrate, potassium nitrate, etc.; an organic nitrogen source such as polypeptone, peptone, meat extract, etc.; or a mixture thereof. Usually, the nitrogen source can be added to the medium in a concentration of about 0.5 to 5 g/l. In addition, an inorganic salt such as dipotassium hydrogen phosphate, potassium dihydrogen phosphate, magnesium sulfate, etc. or a mixture thereof can be added to the medium.

Conditions for cultivation are not limited to specific ones but, usually, cultivation can be carried out in a shaking or submerged culture of pH about 7 to 9.5 at about 25 to 35°C for about 10 hours to 7 days to produce and accumulate 12α-hydroxy ADD or 12β-hydroxy ADD in the medium.

12-Hydroxy ADD thus accumulated in the medium can be collected by making the culture medium basic and extracting the product with an organic solvent, which dissolves 12-hydroxy ADD and is immiscible with water, such as ethyl acetate, chloroform, a mixture solvent of chloroform and methanol, etc. The extraction with the organic solvent can be effected on the culture medium containing microbial cells itself or on a filtrate or supernatent obtained by previously removing microbial cells and other insoluble materials from the culture medium by a known method such as filtration or centrifugation. When the extraction is effected on a filtrate or supernatant, if necessary, the resulting insoluble materials are further extracted with the above organic solvent to recover 12-hydroxy ADD which has been deposited or precipitated in the culture medium. By combining the extracts thus obtained and distilling off the solvent therefrom, almost all the desired 12-hydroxy ADD can be collected.

The resulting extract little contains the remaining substrate and by-products and, therefore, 12-hydroxy ADD can be obtained in high purity by distilling off the solvent from the extract and recrystallizing the residue from, for example, ethyl acetate, dichloromethane-methanol or the like.

12-Hydroxy ADD obtained according to the process of the present invention can be converted into 4-androsten-3,17-dione which is a useful starting material for the synthesis of steroid diuretics such as spironolactone by partially hydrogenating A ring thereof and chemically removing the hydroxy substituent at 12-position.

The following examples further illustrate the present invention in detail but are not to be construed to limit the scope thereof.

Preparation of *Pseudomonas putida* D4014—A1099 strain

One loopful of *Pseudomonas putida* D4014 strain cultivated on a slant medium (medium A: deoxycholic acid 0.5 %, sodium hydroxide 0.05 %, peptone 0.5 %, yeast extract 0.5 %, sodium chloride 0.5 % and agar 1.5 %) was inoculated into a medium (10 ml, medium B: deoxycholic acid 2 %, sodium hydroxide 0.2 %, ammonium nitrate 0.2 %, potassium dihydrogen phosphate 0.1 %, dipotassium hydrogen phosphate 0.6 %, magnesium sulfate heptahydrate 0.02 % and yeast extract 0.02 %) in a test tube (200 mm×21 mm in diameter) and incubated with shaking at 30°C for 14 to 15 hours. The resulting culture (0.3 ml) was added to a medium (10 ml, medium C: deoxycholic acid 0.5 %, sodium hydroxide 0.05 %, glucose 0.1 %, ammonium nitrate 0.2 %, potassium dihydrogen phosphate 0.1 %, dipotassium hydrogen

5

phosphate 0.6 %, magnesium sulfate heptahydrate 0.02 % and yeast extract 0.02 %) in a test tube (200 mm×21 mm in diameter) and incubated at 30°C for 8 to 9 hours. The microbial cells in a log phase were collected by a membrane filter (pore size 0.45 μ) under aseptic conditions, washed with 0.1 M phosphate buffer (pH 7.0, 20 ml) and suspended in the same buffer (25 ml).

Mutagenic treatment was carried out by adding N-methyl-N'-nitro-N-nitrosoguanidine in a final concentration of 50 μg/ml to the above-prepared cell suspension and allowing the resulting mixture to stand for 3 to 4 minutes.

The cells thus treated were collected by a membrane filter (pore size 0.45 μ), washed with 0.1 M phosphate buffer (pH 7.0, 20 ml) and suspended in the same buffer (20 ml). The resulting cell suspension was diluted with a sterilized physiological saline solution and spread on agar plates (medium D: deoxycholic acid 0.5 %, sodium hydroxide 0.05 %, ammonium nitrate 0.2 %, potassium dihydrogen phosphate 0.1 %, dipotassium hydrogen phosphate 0.6 %, magnesium sulfate heptahydrate 0.02 %, yeast extract 0.02 % and agar 1.5 %) so as to form 500 to 1000 colonies per one plate. The plates were incubated at 30°C for 3 to 4 days.

Among the colonies thus formed, a pin point colony was isolated by cultivating it on a slant of the medium A and one loopful of the culture was inoculated into a medium (10 ml, medium E: deoxycholic acid 0.2%, sodium hydroxide 0.02%, glucose 0.1%, ammonium nitrate 0.2%, potassium dihydrogen phosphate 0.1 %, dipotassium hydrogen phosphate 0.6 %, magnesium sulfate heptahydrate 0.02 % and yeast extract 0.02 %) in a test tube (200 mm×21 mm in diameter) and incubated with shaking at 30°C for 24 hours.

Upon examining products accumulated in the medium E by thin layer chromatography, a microbe which selectively produced 12α-hydroxy ADD was found and named *Pseudomonas putida* D4014—A1099 strain.

Example 1

*Pseudomonas putida* D4014—A1099 strain was cultivated as follows:

Composition of culture medium

| | |
|---|---|
| Deoxycholic acid | 0.5 g |
| Glucose | 0.1 g |
| Ammonium nitrate | 0.2 g |
| Potassium dihydrogen phosphate | 0.1 g |
| Dipotassium hydrogen phosphate | 0.6 g |
| Magnesium sulfate heptahydrate | 0.02 g |
| Yeast extract | 0.02 g |
| Sodium hydroxide | 0.05 g |
| Tap water | to 100 ml |

The above ingredients were admixed to obtain a culture medium (pH 8.4, 100 ml). The medium was placed in a Sakaguchi flask (volume 500 ml) and autoclaved at 120°C for 15 minutes. To the flask, there was added seed culture (10 ml) which was obtained by previously cultivating the strain in the same medium with shaking at 30°C for 15 hours. The flask was incubated on a shaker at 30°C for 28 hours.

After completion of culvitation, the culture medium was collected and centrifuged to remove microbial cells. The resulting supernatant was adjusted to about pH 12 by addition of 1 N aqueous solution of sodium hydroxide. The supernatant was extracted with ethyl acetate (300 ml) and the solvent was distilled off from the extract with a rotary evaporator to obtain 12α-hydroxy ADD (360 mg).

Methanol was added to a small portion of 12α-hydroxy ADD thus obtained to prepare 2 % solution. The solution (25 μl) was injected to a high-performance liquid chromatography apparatus equipped with a μBondapak C-18 column (HLC-GPC-244 type manufactured by Waters Associates in U.S.A.). The column was eluted with water-methanol (30:70 v/v, pH 4.0) at the rate of 1 ml/min. and refractive index of the eluate was measured.

The area ratio of each peak of the resulting chromatogram was measured by using a planimeter (Shimadzu Chromatopack C-RIA manufactured by Shimadzu Corporation in Japan). When purity of 12α-hydroxy ADD obtained was determined based on the area ratio, it was 99.5 %.

12α-Hydroxy ADD thus obtained was identified as follows:

Mass spectrum: $m/z=300[M]^{+\cdot}$; and 282 $[M—H_2O]^{+\cdot}$. The presence of 3-keto-1,4-dien was confirmed by $m/z=121$ and 122.

NMR spectrum (90 MHz): $\delta^{DMSO-d6}_{HMS}=0.75$ (3H, s) 18—CH$_3$; 1.13 (3H, s) 19—CH$_3$; 3.85 (1H, d) 12β—H; 4.55 (1H, d) 12α—OH; 5.95 (1H) 4—H; 6.15 (1H, d) 2—H; and 7.10 (1H, d) 1—H.

Example 2

*Pseudomonas putida* D4014—A1099 strain was cultivated according to the same procedure as described in Example 1 except that the culture medium having the following composition (pH 8.4) was used and cultivation was carried out for 6 days.

Composition of culture medium

| | |
|---|---|
| Deoxycholic acid | 2 g |
| Glucose | 0.1 g |
| Ammonium nitrate | 0.2 g |
| Potassium dihydrogen phosphate | 0.1 g |
| Dipotassium hydrogen phosphate | 0.6 g |
| Magnesium sulfate heptahydrate | 0.02 g |
| Yeast extract | 0.02 g |
| Sodium hydroxide | 0.2 g |
| Tap water | to 100 ml |

After completion of cultivation, the culture medium (pH 9.3) was extracted with ethyl acetate (200 ml). The extract was washed with 10 % aqueous solution of sodium bicarbonate (50 ml) and the solvent was distilled off from the extract with a rotary evaporator to obtain 12α-hydroxy ADD (0.46 g).

When purity of 12α-hydroxy ADD thus obtained was determined according to the same procedure as described in Example 1, it was 97.8 %.

Example 3

*Pseudomonas putida* D4014—A1099 strain was cultivated as follows:

Tap water was added to a mixture of deoxycholic acid (25 g), sodium hydroxide (2.5 g), ammonium nitrate (10 g), potassium dihydrogen phosphate (5 g), dipotassium hydrogen phosphate (30 g), magnesium sulfate heptahydrate (1 g) and yeast extract (1 g) and the volume was adjusted to 4.5 liters (pH 8.4). The resulting solution was placed in a fermentation tank (volume 10 liters) and autoclaved at 120°C for 15 minutes. After cooling, a solution of glucose (5 g) in tap water (300 ml) which was separately sterilized was added into the tank to prepare a culture medium. Into the tank, there was added seed culture (300 ml) which was obtained by previously cultivating the strain in the same medium with shaking at 30°C for 10 hours and cultivation was carried out with stirring (300 r.p.m.) and aeration (0.5 VVm) at 30°C for 28 hours.

After completion of cultivation, crystals deposited in the culture medium were collected by decantation, washed several times with water, filtered off with glass filter and dried to obtain 12α-hydroxy ADD (7.4 g). On the other hand, the culture medium separated by decantation was subjected to centrifugation to remove microbial cells. The resulting supernatant (5.5 liters) was adjusted to pH 9.5 by addition of 1 N aqueous solution of sodium hydroxide. The supernatant was extracted twice with ethyl acetate (5.5 liters) and the solvent was distilled off from the extract with a rotary evaporator to obtain 12α-hydroxy ADD (9.2 g).

When their purities were determined according to the same procedure as described in Example 1, they were 99.7 % (in case of 12α-hydroxy ADD deposited in the culture medium) and 96.5 % (in case of 12α-hydroxy ADD obtained from the supernatant).

Example 4

*Pseudomonas putida* D4014 strain was cultivated as follows:

A mixture of deoxycholic acid (1.0 g), glucose (0.1 g), ammonium nitrate (0.2 g), potassium dihydrogen phosphate (0.1 g), dipotassium hydrogen phosphate (0.6 g), magnesium sulfate heptahydrate (0.02 g) and yeast extract (0.05 g) was added to tap water (50 ml) and the resulting solution was adjusted to pH 8.5 by addition of 2 N aqueous solution of sodium hydroxide. The volume of the solution was adjusted to 100 ml with tap water to obtain a culture medium. The culture medium was placed in a Shakaguchi flask (volume 500 ml) and autoclaved at 120°C for 15 minutes. Into the flask, there was added seed culture (4 ml) which was obtained by previously cultivating the strain in the same medium with shaking at 30°C for 15 hours and cultivation was carried out with shaking at 30°C for 48 hours.

When a sample (5 μl) of the resulting culture medium was spotted on a silica gel TLC plate and developed to a distance of 8 cm with isooctane-ethyl acetate-acetic acid (5:5:1, v/v) and, after drying, color was developed by spraying sulfuric acid-ethanol (1:1 v/v) and heating at 120 to 130°C for 5 to 10 minutes, only one clear orange spot at Rf 0.24 was observed. Therefore, it was judged that only one fermentation product was accumulated in the culture medium.

The resulting culture medium was adjusted to about pH 12 by addition of 1N aqueous solution of sodium hydroxide and extracted with ethyl acetate (300 ml). The solvent was distilled off from the extract with a rotary evaporator to obtain a fermentation product (322 mg) which was identified as 12β-hydroxy ADD by the following analyses.

Mass spectrum: m/z=300 $[M]^{+\cdot}$; and 282 $[M—H_2O]^{+\cdot}$. The presence of 3-keto-1,4-dien was confirmed by m/z=121 and 122.

NMR spectrum (90 MHz): $\delta_{TMS}^{CDCl_3}$=0.96 (3H, s) 18—$CH_3$; 1.21 (3H, s) 19—$CH_3$; 3.72 (1H, dd, J=5Hz and 10Hz) 12α—H; 6.06 (1H, s) 4—H; 6.20 (1H, d, J=10Hz) 2—H; and 6.98 (1H, d, J=10Hz) 1—H.

Methanol was added to a small portion of 12β-hydroxy ADD thus obtained to prepare 2 % solution. The solution (25 μl) was injected to a high-performance liquid chromatography apparatus equipped with a μBondapak C-18 column (HLC-GPC-244 type manufactured by Waters Associates in U.S.A.). The column

7

was eluted with water-methanol (30:70, v/v, pH 4.0) at the rate of 1 ml/min. and refractive index of the eluate was measured.

The area ratio of each peak of the resulting chromatogram was measured by using a planimeter (Shimadzu Chromatopack C-RIA manufactured by Shimadzu Corporation in Japan). When purity of 12β-hydroxy ADD obtained was determined based on the area ratio, it was 94.9 %.

## Claims

1. A microbial process for producing 12 - hydroxyandrosta - 1,4 - dien - 3,17 - dione which comprises cultivating a microbe of the species *Pseudomonas putida,* which is capable of producing 12 - hydroxyandrosta - 1,4 - dien - 3,17 - dione by utilizing deoxycholic acid or a salt thereof as a substrate, in a culture medium containing the substrate to produce 12 - hydroxyandrosta - 1,4 - dien - 3,17 - dione and collecting the product.

2. A microbial process according to claim 1, wherein the microbe is *Pseudomonas putida* D4014 strain (FERM BP-205).

3. A microbial process according to claim 2, wherein the product is 12β - hydroxyandrosta - 1,4 - dien - 3,17 - dione.

4. A microbial process according to claim 1, wherein the microbe is *Pseudomonas putida* D4014—A1099 strain (FERM BP-207).

5. A microbial process according to claim 4, wherein the product is 12α - hydroxyandrosta - 1,4 - dien - 3,17 - dione.

6. A microbial process according to claim 1, 2, 3, 4 or 5, wherein the cultivation is carried out under aerobic conditions by using the medium containing the substrate in an amount of 1 to 100 g/l as deoxycholic acid.

## Patentansprüche

1. Mikrobiologisches Verfahren zur Herstellung von 12 - Hydroxyandrosta - 1,4 - dien - 3,17 - dion, gekennzeichnet durch die Kultivierung von Mikroben der Art Pseudomonas putida, die 12 - Hydroxyandrosta - 1,4 - dien - 3,17 - dion durch Verwertung von Desoxycholinsäure oder eines ihrer Salze als Substrat produzieren können, in einem das Substrat enthaltendem Kulturmedium zur Herstellung von 12 - Hydroxyandrosta - 1,4 - dien - 3,17 - dion und das Sammeln des Produkts.

2. Mikrobiologisches Verfahren nach Anspruch 1, gekennzeichnet durch die Verwendung des Pseudomonas-putida-Stammes D4014 (FERM BP 205) als Mikroben.

3. Mikrobiologisches Verfahren nach Anspruch 2, gekennzeichnet, durch die Gewinnung von 12β - Hydroxyandrosta - 1,4 - dien - 3,17 - dion als Produkt.

4. Mikrobiologisches Verfahren nach Anspruch 1, gekennzeichnet durch die Verwendung des Stammes Pseudomonas-putida D4014—A1099 (FERM BP 207) als Mikroben.

5. Mikrobiologisches Verfahren nach Anspruch 4, gekennzeichnet durch die Gewinnung von 12α - Hydroxyandrosta - 1,4 - dien - 3,17 - dion als Produkt.

6. Mikrobiologisches Verfahren nach Anspruch 1, 2, 3, 4 oder 5, gekennzeichnet durch die Durchführung der Kultivierung in einer Menge von 1 bis 100 g/l als Desoxycholinsäure enthaltenden Mediums.

## Revendications

1. Procédé microbiologique pour préparer la 12 - hydroxyandrosta - 1,4 - diène - 3,17 - dione, qui consiste à cultiver un microorganisme de l'espèce *Pseudomonas putida* qui est capable de produire la 12 - hydroxyandrosta - 1,4 - diène - 3,17 - dione en utilisant l'acide désoxycholique ou un de ses sels comme substrat dans un milieu de culture contenant le substrat pour produire la 12 - hydroxyandrosta - 1,4 - diène - 3,17 - dione et à recueillir le produit.

2. Procédé microbiologique selon la revendication 1, dans lequel le micro-organisme est la souche *Pseudomonas putida* D4014 (FERM BP-205).

3. Procédé microbiologique selon la revendication 2, dans lequel le produit est la 12β - hydroxyandrosta - 1,4 - diène - 3,17 - dione.

4. Procédé microbiologique selon la revendication 1, dans lequel le micro-organisme est la souche *Pseudomonas putida* D4014—A1099 (FERM BP-207).

5. Procédé microbiologique selon la revendication 4, dans lequel le produit est la 12α - hydroxyandrosta - 1,4 - diène - 3,17 - dione.

6. Procédé microbiologique selon la revendication 1, 2, 3, 4 ou 5, dans lequel la culture est mise en oeuvre en conditions aérobies en utilisant un milieu contenant le substrat en quantité de 1 à 100 g/l, calculé en acide désoxycholique.